# EUROPEAN PATENT SPECIFICATION

(11) **EP 2 002 803 B1**
(45) Date of publication and mention of the grant of the patent: **22.12.2010**
(21) Application number: 08102185.9
(22) Date of filing: 29.02.2008
(51) Int. Cl.: A61F 2/16

(54) **Lens injector lumen tip for wound assisted delivery**
Lumenspitze mit Linseneinspritzung für die Wundenversorgung
Extrémité de lumière d'injecteur de lentille pour une mise en place assistée dans la plaie

(30) Priority: 12.06.2007 US 761457
(43) Date of publication of application: 17.12.2008
(73) Proprietor: Alcon, Inc., 6331 Hünenberg (CH)
(72) Inventor: Downer, David A., Fort Worth, TX 76137 (US); Tjia, Khiun F., Epe 8162 MA (NL)
(74) Representative: Hanna, Peter William Derek

(56) References cited:
- EP-A- 1 857 076
- WO-A-02/083216
- US-A- 5 195 526
- US-A1- 2006 167 466
- US-A1- 2006 200 167

## Description

This invention relates to intraocular lenses (IOLs) and more particularly to cartridges used to inject IOLs into an eye.

### Background of the Invention

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens.

When trauma, age or disease cause the lens to become less transparent, vision deteriorates because of the diminished light which can be transmitted to the retina. This deficiency in the lens of the eye is medically known as a cataract. The treatment for this condition is surgical removal of the lens and implantation of an artificial lens or IOL.

While early IOLs were made from hard plastic, such as polymethylmethacrylate (PMMA), soft, foldable IOLs made from silicone, soft acrylics and hydrogels have become increasingly popular because of the ability to fold or roll these soft lenses and insert them through a smaller incision. Several methods of rolling or folding the lenses are used. One popular method is an injector cartridge that folds the lenses and provides a relatively small diameter lumen through which the lens may be pushed into the eye, usually by a soft tip plunger. The most commonly used injector cartridge design is illustrated in U.S. Patent No. 4,681,102 (Bartell), and includes a split, longitudinally hinged cartridge. Similar designs are illustrated in U.S. Patent Nos. 5,494,484 and 5,499,987 (Feingold) and 5,616,148 and 5,620,450 (Eagles, et al.). In an attempt to avoid the claims of U.S. Patent No. 4,681,102, several solid cartridges have been investigated, see for example U.S. Patent No. 5,275,604 (Rheinish, et al.), 5,653,715 (Reich, et al.), and U.S. Patent No. 5,947,976 (Van Noy, et al).

These prior art devices were intended to inject an IOL into the posterior chamber of an aphakic eye through a relatively large (approximately 3.0 mm or larger) incision. Surgical techniques and IOLs have been developed that allow the entire surgical procedure to be performed through much smaller incisions, 2.4 mm and smaller. As a result, surgeons began developing methods of wound assisted IOL insertion, where the IOL is delivered through a small incision without inserting the cartridge tip fully into the wound. In this type of IOL delivery, the wound itself provides a tunnel through which the IOL enters the anterior chamber. Wound assisted IOL delivery, therefore, eliminates the need for the incision to be large enough to accommodate the outer diameter of the cartridge tip, allowing a smaller incision to be used. Prior to the present invention, such wound assisted delivery was accomplished using techniques which are highly reliant on the degree of skill and confidence of the surgeon.

EP-A-1,857,076 (published 21.11.2007) (Canon Staar Co.) describes an IOL insertion device having a plane or chamfered nozzle opening and at least one peripheral protrusion extending laterally from the outer wall of the nozzle proximally from the opening, designed to prevent wound leakage following full insertion of the nozzle in use.

US 2006/167466 (Dusek) and US 2006/200167 (Peterson) also describe an IOL insertion device having a chamfered nozzle opening for full insertion in an incision in use.

Accordingly, a need continues to exist for an intraocular lens injection cartridge which provides features to specifically aid in wound assisted IOL delivery.

### Brief Summary of the Invention

The present invention relates to an intraocular lens injector cartridge according to claim 1. Preferred embodiments of the invention are set forth in the dependent claims.

The present invention improves upon prior art by providing a cartridge for an IOL delivery system that includes an extended canopy at the distal tip of the cartridge to open and support the wound while guiding and controlling the folded lens as it passes through the wound, and a peripheral protrusion, flange, or stop feature that provides an insertion depth limitation and prevention of full insertion of the cartridge tip, in accordance with claims which follow. In addition, the protrusion provides support to the incision to reduce the tendency of wound damage through tearing.

It is accordingly an objective of the present invention to provide a cartridge for a lens delivery system that has an extended canopy at the distal tip.

It is a further objective of the present invention to provide a cartridge for a lens delivery system that contains a peripheral protrusion, flange, or stop that provides an insertion depth limitation.

It is yet a further objective of the present invention to provide a cartridge for a lens delivery system that contains a peripheral protrusion, flange, or stop that provides support to the incision to reduce the tendency of wound damage through tearing.

Other objectives, features and advantages of the present invention will become apparent with reference to the drawings, and the following description of the drawings and claims.

### Brief Description of the Drawings

FIG. 1 is an enlarged top perspective view of the lens delivery system cartridge.
FIG. 2 is an enlarged front partial perspective view of a first embodiment of the distal tip of the lens delivery system cartridge.
FIG. 3 is an enlarged side elevational view of a first embodiment of the distal tip of the lens delivery system cartridge.
FIG. 4 is an enlarged front partial perspective view of a second embodiment of the distal tip of the lens delivery system cartridge of the present invention.
FIG. 5 is an enlarged front elevational view of a third embodiment of the distal tip of the lens delivery system cartridge of the present invention.
FIG. 6 is an enlarged side elevational view of the lens delivery system cartridge inserted into an incision in an eye.

### Detailed Description of the Preferred Embodiments

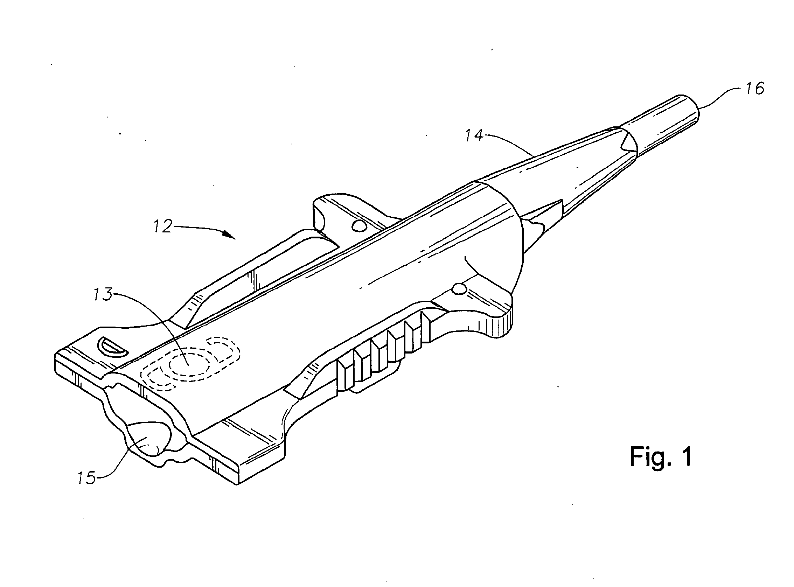

As best seen in FIG. 1, lens cartridge 10 of the present invention generally includes body 12 and nozzle 14. Cartridge 10 can be molded from any suitable thermoplastic, such as polypropylene, and the thermoplastic may contain a lubricity enhancing agent such as those disclosed in U.S. Pat. No. 5,716,364. Nozzle 14 may be integrally formed with body 12. Nozzle 14 includes distal tip 16. Body 12 contains bore or lumen 15. Prior to use, IOL 13 is initially positioned within lumen 15 of body 12.

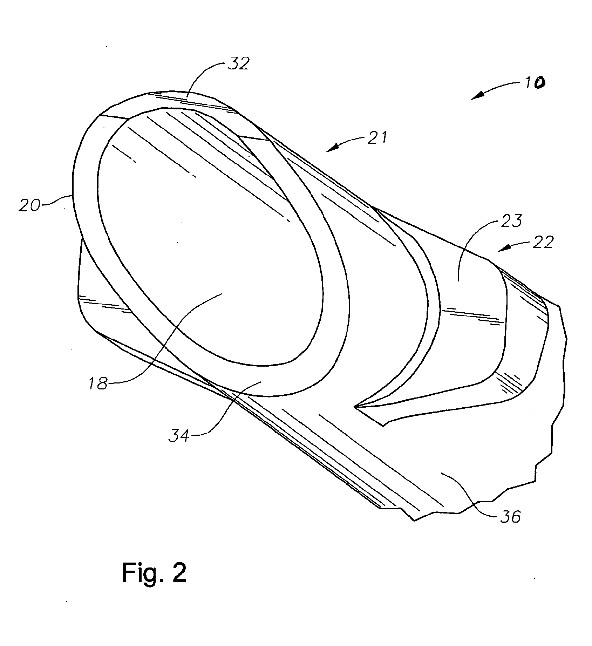

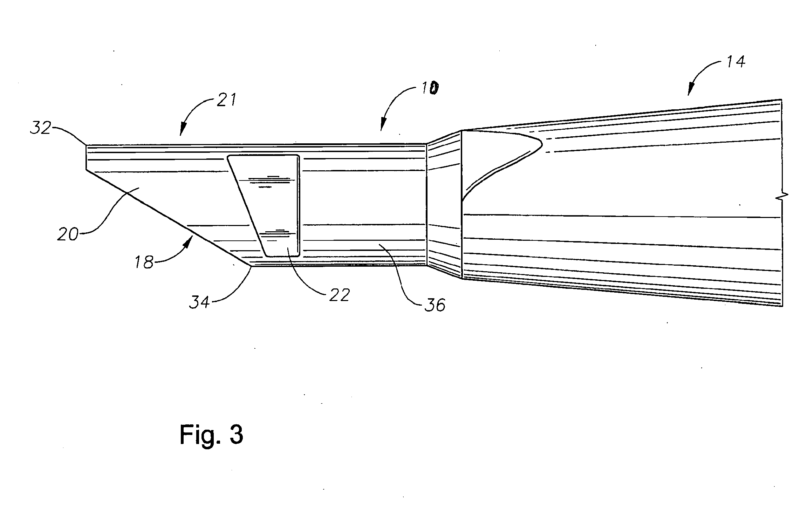

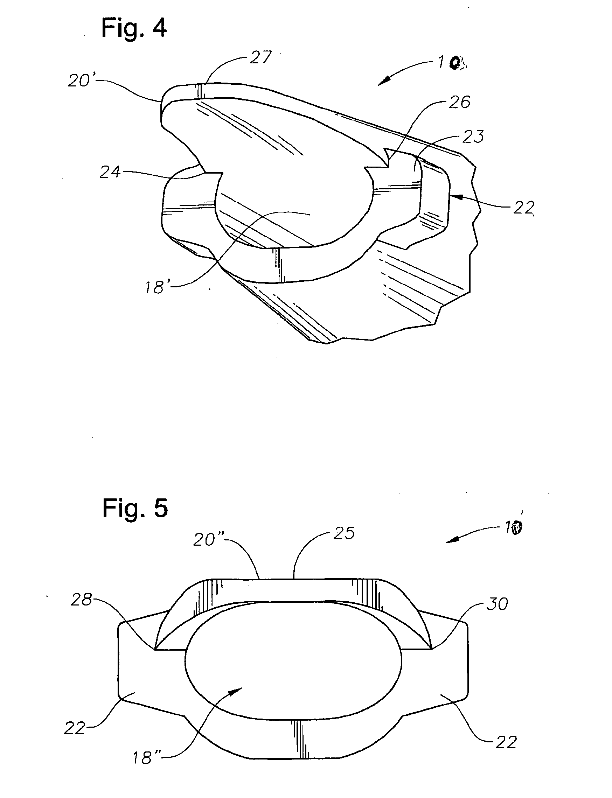

As best seen in FIGS. 2-3, distal tip 16 includes opening 18, canopy 20, and peripheral protrusion 22. Canopy 20 extends from distal tip 16 and functions to open the wound prior to IOL 13 insertion. In addition, upper portion 21 of canopy 20 provides support for the folded IOL 13 during wound assisted delivery. In a preferred embodiment, illustrated in FIGS. 2 and 3, canopy 20 completely encircles opening 18. In this embodiment, canopy 20 gradually tapers from its maximum length of between approximately 1.5 millimeters to 2.4 millimeters (preferably about 1.9 millimeters) at approximately 12 o'clock position 32 to flush with opening 18 at approximately 6 o'clock position 34. In a second embodiment, shown in FIG. 4, canopy 20' only partially encircles opening 18' from approximately 10 o'clock position 24 to approximately 2 o'clock position 26. Similarly to canopy 20, canopy 20' gradually tapers from a 5 maximum length of between approximately 1.5 millimeters to 2.4 millimeters (and preferably about 1.9 millimeters), at position 27, to flush with opening 18' at approximately 10 o'clock position 24 and approximately 2 o'clock position 26. In a third embodiment, shown in FIG. 5, canopy 20" substantially encircles opening 18" from approximately 9 o'clock position 28 to approximately 3 o'clock position 30. In this embodiment, canopy 20" also gradually tapers from a maximum length of between approximately 1.5 millimeters to 2.4 millimeters (also preferably about 1.9 millimeters), at position 25, to flush with opening 18" at approximately 9 o'clock position 28 and approximately 3 o'clock position 30.

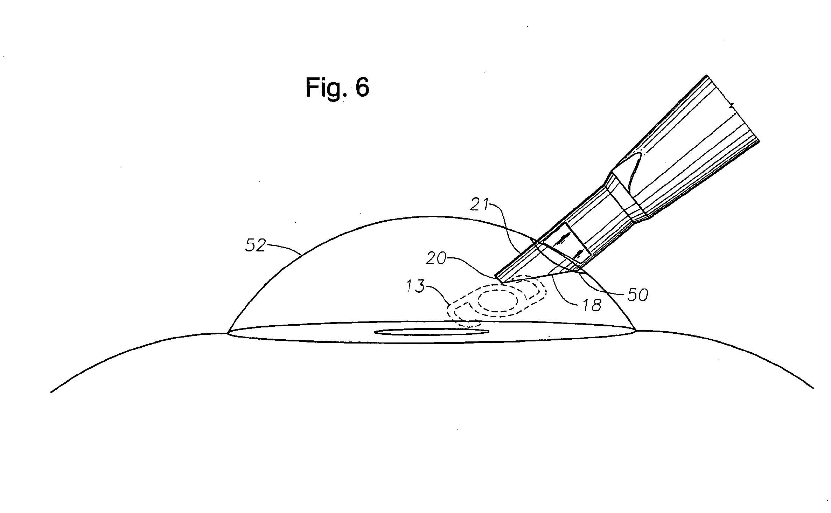

Peripheral protrusion 22 may be any feature appropriate for preventing distal tip16 from fully entering an incision, such as a flange or stop. Peripheral protrusion 22 extends laterally from outer wall 36 of distal tip 16, and may be a continuous protrusion that completely encircles nozzle 14. However, peripheral protrusion 22 may not be continuous and most preferably comprise a plurality of protrusions 22 that extend, for example, laterally from either side of outer wall 36 of distal tip 16. Peripheral protrusion 22 serves as an insertion depth limitation, and prevents the full insertion of distal tip 16 into the wound entrance. Distal face 23, of peripheral protrusion 22 may be square or sloped at an angle of between approximately 18 to 26 degrees (preferably about 22 degrees). Such a slope will allow a more contoured contact with the surface of the eye and provide for less tissue irritation because cartridge 10 generally is held at an angle to eye 52 during use, as seen in FIG. 6. Both canopy 20 and peripheral protrusion 22 may be integrally formed with distal tip 16 and nozzle 14.

During operation, shown in FIG. 6, the surgeon makes incision 50 in of eye 52. Canopy 20 is inserted into incision 50 to such a depth where peripheral protrusion 22 contacts the surface of eye 52 and prevents further insertion. Upper portion 21 of canopy 20 holds incision 50 open and supports IOL 13 as it is moved through opening 18, through incision 50, and inserted into eye 52.

While certain embodiments of the present invention have been described above, these descriptions are given for purposes of illustration and explanation.

## Claims

1. An intraocular lens injector cartridge (10), comprising:
a) a body (12) having an internal lumen (15);
b) a tubular nozzle (14) having an outer wall (36) and an opening (18), the nozzle projecting distally from the body, the opening being fluidly connected to the internal lumen of the body;
c) at least one peripheral protrusion (22) extending laterally from the outer wall of the nozzle proximally from the opening;
**characterized in that**
the at least one peripheral protrusion (22) is spaced proximally from the plane of the opening so as to provide an insertion depth limitation and prevent full insertion of the cartridge tip, in use, and wherein the nozzle opening is defined by an extended canopy (20, 20', 20") projecting distally from a plane of the opening (18, 18', 18") at least partially encircling the opening.

2. The cartridge of claim 1, wherein the canopy (20') partially surrounds the opening (18') between approximately a 10 o'clock position to approximately a 2 o'clock position.

3. The cartridge of claim 1, wherein the canopy (20") partially surrounds the opening (18") between approximately a 9 o'clock position to approximately a 3 o'clock position.

4. The cartridge of claim 1, wherein the canopy (20) entirely surrounds the opening (18).

5. The cartridge of any one of claims 1 to 4, wherein the peripheral protrusion (22) comprises a plurality of protrusions.

6. The cartridge of any one of claims 1 to 4, wherein the peripheral protrusion (22) comprises a continuous protrusion encircling the nozzle (14).

7. The cartridge of any one of claims 1 to 6, wherein the peripheral protrusion (22) comprises an angled distal face (23).

8. The cartridge of any one of claims 1 to 6, wherein the peripheral protrusion (22) comprises a flange.

9. The cartridge of any one of claims 1 to 6, wherein the peripheral protrusion (22) comprises a stop.

## Patentansprüche

1. Intraokularlinsen-Einfiihrpatrone (10), die aufweist:
a) einen Körper (12) mit einem internen Lumen (15);
b) eine röhrenförmige Düse (14) mit einer Außenwand (16) und einer Öffnung (18), wobei die Düse distal von dem Körper vorsteht, wobei die Öffnung in Fluidverbindung mit dem internen Lumen des Körpers steht;
c) wenigstens einen peripheren Vorsprung (22), der sich seitlich von der Außenwand der Düse proximal von der Öffnung erstreckt;
**dadurch gekennzeichnet, dass**
der wenigstens eine periphere Vorsprung (22) proximal von der Ebene der Öffnung beabstandet ist, um eine Begrenzung der Einführtiefe bereit zu stellen und im Gebrauch die komplette Einführung der Patronenspitze zu verhindern, und wobei die Düsenöffnung durch ein erweitertes Dach (20, 20', 20") definiert ist, das distal von einer Ebene der Öffnung (18, 18', 18") vorsteht und die Öffnung wenigstens teilweise umgibt.

2. Patrone nach Anspruch 1, wobei das Dach (20') die Öffnung (18') zwischen etwa einer 10-Uhr-Position und etwa einer 2-Uhr-Position teilweise umgibt.

3. Patrone nach Anspruch 1, wobei das Dach (20") die Öffnung (18") zwischen etwa einer 9-Uhr-Position und etwa einer 3-Uhr-Position teilweise umgibt.

4. Patrone nach Anspruch 1, wobei das Dach (20) die Öffnung (18) vollkommen umgibt.

5. Patrone nach einem der Ansprüche 1 bis 4, wobei der periphere Vorsprung (22) eine Vielzahl von Vorsprüngen aufweist.

6. Patrone nach einem der Ansprüche 1 bis 4, wobei der periphere Vorsprung (22) einen kontinuierlichen Vorsprung aufweist, der die Düse (14) umgibt.

7. Patrone nach einem der Ansprüche 1 bis 6, wobei der periphere Vorsprung (22) eine abgewinkelte distale Fläche (23) aufweist.

8. Patrone nach einem der Ansprüche 1 bis 6, wobei der periphere Vorsprung (22) einen Flansch aufweist.

9. Patrone nach einem der Ansprüche 1 bis 6, wobei der periphere Vorsprung (22) einen Anschlag aufweist.

## Revendications

1. Cartouche d'injecteur de cristallin artificiel (10), comprenant :
a) un corps (12) ayant une lumière interne (15) ;
b) une buse tubulaire (14) ayant une paroi externe (36) et une ouverture (18), la buse faisant distalement saillie du corps, l'ouverture étant raccordée de manière fluide à la lumière interne du corps ;
c) au moins une saillie périphérique (22) s'étendant latéralement à partir de la paroi externe de la buse de manière proximale par rapport à l'ouverture ;
**caractérisée en ce qui** :
la au moins une saillie périphérique (22) est espacée de manière proximale par rapport au plan de l'ouverture de fournir une limitation de profondeur d'insertion et empêcher l'insertion totale de l'embout de cartouche, à l'usage, et dans laquelle l'ouverture de buse est définie par un couvercle étendu (20, 20', 20") faisant saillie de manière distale par rapport à un plan de l'ouverture (18, 18', 18") encerclant au moins partiellement l'ouverture.

2. Cartouche selon la revendication 1, dans laquelle le couvercle (20') entoure partiellement l'ouverture (18') entre approximativement une position à 10 heures jusqu'à une position approximativement à 2 heures.

3. Cartouche selon la revendication 1, dans laquelle le couvercle (20") entoure partiellement l'ouverture (18") entre approximativement une position à 9 heures jusqu'à approximativement une position à 3 heures.

4. Cartouche selon la revendication 1, dans laquelle le couvercle (20) entoure entièrement l'ouverture (18).

5. Cartouche selon l'une quelconque des revendications 1 à 4, dans laquelle la saille périphérique (22) comprend une pluralité de saillies.

6. Cartouche selon l'une quelconque des revendications 1 à 4, dans laquelle la saillie périphérique (22) comprend une saillie continue encerclant la buse (14).

7. Cartouche selon l'une quelconque des revendications 1 à 6, dans laquelle la saillie périphérique (22) comprend une face distale coudée (23).

8. Cartouche selon l'une quelconque des revendications 1 à 6, dans laquelle la saillie périphérique (22) comprend un rebord.

9. Cartouche selon l'une quelconque des revendications 1 à 6, dans laquelle la saillie périphérique (22) comprend une butée.
